# Europäisches Patentamt

## European Patent Office

## Office européen des brevets

(11) Publication number: **0 086 179**
**B1**

(12) **EUROPEAN PATENT SPECIFICATION**

(45) Date of publication of patent specification: **01.06.88**

(51) Int. Cl.⁴: **C 12 N 11/04**, C 12 P 19/14, C 12 N 11/08, C 12 N 11/12 // C12N9/38

(21) Application number: **83830022.6**

(22) Date of filing: **02.02.83**

(54) **Capillary filter consisting of semipermeable membrane with enzymatic activity, process for its manufacture and its applications.**

(30) Priority: **04.02.82 IT 4772082**

(43) Date of publication of application:
**17.08.83 Bulletin 83/33**

(45) Publication of the grant of the patent:
**01.06.88 Bulletin 88/22**

(84) Designated Contracting States:
**CH DE FR GB LI NL SE**

(56) References cited:

**ULLMANS ENCYKLOPÄDIE DER TECHNISCHEN CHEMIE, vol. 16, 4th edition, 1978, pages 515-535, E. Bartholomé, Weinheim, DE E. STAUDE: "Membranen"**

**CARBOHYDRATE RESEARCH, vol. 8, 1968, pages 491-497, Elsevier Publishing Company, Amsterdam, printed in Belgium S.A. BARKER et al.: "Preparation and properties of alpha-amylase chemically coupled to microcrystalline cellulose"**

(73) Proprietor: **CONSIGLIO NAZIONALE DELLE RICERCHE**
**Piazzale Aldo Moro, 7**
**I-00185 Roma (IT)**

(72) Inventor: **Drioli, Enrico**
**Via Aniello Falcone 153**
**Napoli (IT)**
Inventor: **de Rosa, Mario**
**Via E. Nicolardi 188**
**Napoli (IT)**
Inventor: **Gambacorta, Agata**
**Via Bernardo Cavallino 70**
**Napoli (IT)**
Inventor: **Nicolaus, Barbara**
**Rampe Brancaccio 9**
**Napoli (IT)**
Inventor: **Iorio, Gabriele**
**Via S. Francesco 211**
**Maddaloni (Caserta) (IT)**
Inventor: **Punzo, Alfredo**
**Via Giovanni Amendola 6**
**Portici (Napoli) (IT)**

(74) Representative: **Bazzichelli, Alfredo et al**
**c/o Società Italiana Brevetti S.p.A. Piazza Poli 42**
**I-00187 Roma (IT)**

Courier Press, Leamington Spa, England.

(58) References cited:

CHEMICAL ABSTRACTS, vol. 95, part 1, 6th July 1981, page 277, no. 2647b, Columbus, Ohio, US V. BUONOCORE et al.: "A constitutive beta-galactosidase from the extreme thermoacidophile archaebacterium Caldariella acidophila: properties of the enzyme in the free state and in immobilized whole cells"

CHEMICAL ABSTRACTS, vol. 96, part 13, 29th March 1982, page 559, no. 102473f, Columbus, Ohio, US E. DRIOLI et al.: "Whole cell immobilization in polyurethane structural foam"

**0 086 179**

**Description**

The present invention refers to a filter for ultrafiltration, reverse osmosis, dialysis and the like, whose filtrant elements consist of capillaries formed from semipermeable membranes of polymeric material, with a microbial cell immobilized in it whose cell is permeabilized and whose enzymatic activity is completely maintained.

The invention also refers to a process for manufacturing the filter and to its applications for the production of molecular species which underwent a biocatalytic transformation on passing through the enzymatic membranes.

Separation processes based on the use of artificial polymeric membranes, such as reverse osmosis and ultrafiltration, for example, are becoming ever more important industrially, because of their low energy consumption and their versatility of use.

The possibility of realizing these separation processes at the industrial level is related above all to the existence of semipermeable membranes characterized by suitable transport and mechanical properties, as well as by good chemical and thermal resistance. In practice, all membranes currently available commercially consist of polymeric materials. A technique used to prepare this type of membrane is commonly known as the phase inversion technique, and may be used to prepare membranes starting from all polymers which may be used to prepare a homogeneous solution with suitable solvents, from which the polymer can subsequently be precipitated in the continuous phase form.

Membrane formation can be seen as a phase separation process in which a homogeneous polymeric solution is transformed into a biphasic system, consisting of a solid phase rich in polymer which forms the structure of the membrane and of a liquid phase poor in polymer, which forms the pores of the membrane (see, for example, H. Strathmann, K. Koch "The formation mechanism of phase inversion membranes" Desalination (1977)).

The semipermeable membrane can assume various shapes in its mounting in a filter. There are currently three types of shape known, that is: flat, tubular and hollow capillary or hollow fiber.

Membranes in the shape of hollow fibers generally have lower permeability than flat or tubular membranes: in any case, the packing density is high enough to compensate for the inconveniences deriving from the lower permeability. The hollow fibers have characteristic dimenesions on the order of 45 μm outer diameter and 24 μm inner diameter. The ratio of the outer diameter to the thickness of the wall is sufficiently small for the fibers to withstand high external pressure.

The hollow fibers or capillaries may be prepared by means of the dry-wet spinning method.

·According to this method, a polymer solution is pushed through an extruder, and undergoes phase inversion at the outlet upon exposure to the air. The extruded material is then passed into a coagulatory bath kept in rotatory motion, to confer a certain tension upon the fiber during its formation. The inner cavity of the fiber or capillary contains another coagulating fluid to prevent occlusion during its formation. The filter consists of a bundle of capillaries suitably assembled in a cylindrical container, at the ends of which the hollow fibers are joined to one another and to the walls of the container. The solution to be ultrafiltered is brought into the filter under pressure, and the permeate is collected in the space around the outer surface of the capillaries on the inside of the container.

This particular type of geometry allows compact filtrant modules to be formed, with an extremely high surface extension, to allow yields of industrial interest.

Since it already occurs in many biological structures, it would be extremely interesting to use multienzymatic catalysts or systems of catalysts immobilized in the membrane phase in separation processes using semipermeable membranes in capillary form.

Despite the great demand on the part of a broad range of users for biocatalytic systems immobilized in synthetic membranes like those used in ultrafiltration and reverse osmosis processes, it has not yet proved possible to entrap enzymes, multienzymatic systems, bacterial cells and organelles, due to the denaturant conditions upon which the preparation of these synthetic membranes is currently based. In fact, this leads to the use of anhydrous organic solvents and thermal treatments above 60—70°C.

The aim of the present invention is the preparation of synthetic membranes in the form of hollow capillaries, with entrapped biocatalytic activity stable to the organic sovlents and the temperature.

In this way, parallel to the ultrafiltration process, a series of chemical reactions may be carried out at the level of the permeated molecules, thanks to the pressence of the entrapped enzymes in the polymer phase.

It has also been found possible to entrap not only individual enzymes but also whole bacterial cells in polymer membranes in the form of hollow capillaries, prepared by the dry-wet spinning method with the phase inversion system.

This latter methodology is of great interest because it allows enormous reductions in the costs of producing biocatalysts.

Immobilizing a whole microbial cell means blocking a hundred different enzymes in the synthetic membrane, compartmentalized on the inside of the bacterial wall.

For a certain enzymatic activity to be achieved under these conditions, the following points must be observed:

a) the bacterial wall cannot obstruct the flow of substrates and products to and from the enzyme;

3

b) the other biocatalysts present must not be further transformed, with undesired side reactions, substrates and products.

If both these conditions are satisfied, the immobilization of a whole cell rather than the individual purified enzyme, is advantageous not only because it eliminates the complex and costly purification operations, but also because in this way the enzyme remains in an optimal "natural" type microenvironment.

According to the present invention, therefore, during the preparation of the synthetic membrane, the polymer solution is charged with a certain quantity of dehydrated cells of a microorganism resistant to heat and to organic solvents.

Examples of polymers suitable for this preparation include polysulfones, aromatic polyamides, cellulose acetate, acrylonitrile.

A microorganism suitable for charging the membrane is Caldariella acidophila, recently reclassified with the systematic name Sulfolobus solfataricus (see publication 4), an extremely thermophylic bacteria, whose optimal growth temperature may reach 87°C. A strain of the microorganism was deposited in 1974 with an optimal temperature of 75°C called MT-3, at the Torry Research Station, Aberdeen, Great Britain (see publication 1).

The most thermophylic strain (87°C) of Caldariella acidophila, designated MT-4, to which the present discussion refers, has been available since 1980 from the Deutsche Sammlung von Mikroorganismen DSM-Grisebach Strasse 8, D-3400 Göttingen (West Germany), with the name Sulfolobus solfataricus and the deposit number DSM-1617.

The bacteria has been isolated from a volcanic environment near the solfatara of Pozzuoli (Naples), and its physiological and morphological characterization is reported in the following publications:

1) M. DeRosa, A. Gambacorta and J. D. Bu'lock, "Extremely thermophilic acidophilic bacteria convergent with Sulfolobus acidocaldarius", J. Gen. Microbiol., 86 156—164 (1975);

2) G. Millonig. M. DeRosa, A. Gambacorta and J. D. Bu'lock, "Ultrastructure of an extremely thermophilic acidophilic microoganism", J. Gen. Microbiol., 86, 165—173 (1975);

3) M. DeRosa, A. Gambacorta, G. Millonib, J. D. Bu'lock, "Convergent characters of extremely thermophilic acidophilic bacteria", Experentia 30, 866—868 (1974);

4) W. Zillig, K. O. Stetter, S. Wunderl, W. Schulz, H. Pries and I. Scholz, "The Solfolobus—Caldariella" Group: Taxonomy on the basis of the structure of DNA-dependent RNA polymerases", Arch. Microbiol. 125, 259 (1980).

Of the cytoplasmic enzymes of the Caldariella acidophila permeabilized and entrapped in the membrane for ultrafiltration, the following activities were tested: β-galactosidase (EC 3.2.1.23);

methylthioadenosine phosphorylase (EC 2.4.2.1);

glucokinase (EC 2.7.1.2);

glucofructoseisomerase (EC 5.3.1.9);

xylose isomerase (EC 5.3.1.5);

ribose isomerase (EC 5.3.1.6);

maltose dehydrogenase (EC 1.1.1.39).

The behaviour was analyzed in detail, and the applicative potentially was evaluated for hollow fibers of cellulose acetate and polysulfone contained in the polymer phase cells of Calderiella acidophila capable of hydrolyzing lactose into glucose and galactose.

These membranes maintain their biocatalytic capacities through long use in high temperature processes (60—85°C) and are stable on storage, with no particular expedients.

The study showed that:

a) membranes with a different degree of enzymatic activity could be prepared by varying the quantity of filler or bacterial charge;

b) the process for entrapment of the cells in the polymer phase permeabilized the bacterial wall, facilitating passage of the lactose, glucose and galactose through the cytoplasmic membrane; *therefore the enzymatic activity shown by the entrapped cells is 30 to 100 times higher than that of the intact cells*;

c) the products found in the permeate when the lactose is passed on the membrane are mainly glucose and galactose.

The potential of this type of hollow capillary fiber is considerable since they do not contain a single enzyme only, but the entire biocatalytic patrimony of the microorganism, they can thus be used for performing different reactions by modifying the substrates used. One can also hypothesize process schemes in which two or more enzymatic systems are subjected to biocatalysis, separately or in sequence. The latter possibility is of particular interest with regard to processes for in situ regeneration of the coenzymes or cofactors necessary for the realization of a large number of enzymatic reactions.

With regard to the polymer matrix which makes up the wall of the membranes in capillary configuration, in particular the choice of polysulfone gives rise to a system which is stable under the severe operating conditions (60—85°C) used for long periods in this process.

Fiber preparation involves the use of rigorously anhydrous organic systems. Since the bacterial cells which act as filler or packing contain a considerable quantity of water both internally (cytoplasmatic water) and externally (extracellular water), this material must first be dried by repeated treatments with acetone.

4

The organic solvent not only removes the water, but also permeabilizes the bacterial wall, which without losing its mechanical stability, becomes completely permeable to low molecular weight species like glucose, galactose and lactose, respectively the products and substrate of lactase or β-galactosidase, the cytoplasmatic enzyme of the microorganism which is the object of this study.

The cells treated in this way are then washed with the polymer solvent and added to a solution of polymer of said solvent. The suspension of bacterial cells in the polymer solution is then introduced to a dry-wet spinning system which allows preparation of the hollow capillary fibers. The capillary tubes prepared in this way are then washed repeatedly with water and mounted in bundles on the inside of the cylindrical reactors. The ratio by weight of the bacterial cell change to the polymer can reach 1:1, e.g. the ratio can be 1:2 to 1:1.

Example 1
Preparation of the bacteria
A MT-4 strain of Caldariella acidophila was grown at 87°C in a fermentor with slow mechanical agitation and an aeration flux of 2.4—3 l/min. The culture medium contained 1 g/l of yeast extract, 1 g/l casamino acids, 3.1 g/l of $KH_2PO_4$; 2.5 g/l of $(NH_4)_2SO_4$, 0.20 g/l of $MgSO_4 \cdot 7H_2O$, 0.25 g/l of $CaCl_2 \cdot 2H_2O$. The pH was adjusted to 3.0 with 0.1N $H_2SO_4$. The cells were harvested in the logarithmic growth phase at a concentration of 0.5 g of dry cells per liter of culture medium by continuous flow centrifugation in an Alfa-Laval Model LAB 102B-20 separator. The pellet was washed twice in 10 mM Tris-HCl, pH 7.5, and collected by centrifugation at 10,000 rpm for 30 min. Bacteria could be stored at −20°C for months without any loss of enzymatic activity.

To remove the water completely, the cells were washed twice with pure acetone (30% v/v suspension) for at least 30 minutes, recovering the cells by centrifugation. The material treated in this way maintains its biocatalytic properties.

Preparation of polysulfone capillaries
A polymer solution of the following composition (by weight %) was prepared:

| PS | polysulfone, Union Carbide 3500 | 20% |
| PVP | polyvinylpyrrolidone, Merck 25,000—30,000 | 10% |
| DMAC | dimethylacetamide, Carlo Erba | 70%. |

30 g of Calderiella acidophila, prepared and dehydrated as previously described, was suspended in 30 g of dimethylacetamide and added to 300 g of the polymer solution as described above.

The proportions used above correspond to a preparation with a cell to polymer ratio of 1:2.

This suspension was then introduced in a dry-wet spinning system to prepare the hollow capillaries.

Example 2
Preparation of cellulose acetate capillaries
Using essentially the same procedure described in Example 1, a cellulose diacetate was used as polymer, with an acetylation degree of 2.3—2.7 acetylated hydroxyls per glucose unit. A polymer solution of the following composition was prepared (weight%): cellulose acetate 36%, N,N-dimethylformamide 43%, acetone 21%.

12 g of Calderiella acidophila cells prepared as described in the first paragraph of Example 1 were dehydrated with acetone and suspended in 30 g of said solvent, the resulting suspension was added to 70 g of the polymer solution with continuous stirring for approximately 10 minutes to prepare a stable and homogeneous suspension, ready to be introduced in the dry-wet spinning system.

Hollow capillaries containing immobilized Calderiella acidophila cells may be prepared analogously using aromatic polyamide or polyacrylonitrile polymers.

The capillaries produced as described in the preceding examples were tested both for their effectiveness as semipermeable membranes for ultrafiltration, as well as for their biocatalytic conversion reactivity.
Assay of the β-galactosidase entrapped in the hollow fibers
15 cm of capillary containing 50 mg of bacteria were taken from 90 m of capillary in which 30 g of Caldariella acidophila had been entrapped (Example 1). The capillary was cut and suspended in 0.5 ml of 50 mM acetate buffer, pH 5, 100 mM in lactose; the enzymatic reaction was carried out by incubating at 80°C for 5 minutes. The reaction was then quenched in ice, and 200 l were taken for measurement using the Boerhinger glucose-oxidase-peroxidase method.

This test shows that the bacteria contained in the capillaries have double the enzymatic activity of the fresh cells, before permeabilization and entrapment.

Permeability test
Using a lactose solution as described for the preceding assay, and using a capillary produced with the method described in Example 1, permeation was tested by passing an axial flow of 250 ml/min of the solution through the capillary at a temperature of 76°C.

The resulting permeation rate was 24 ml/hour.

Degree of conversion as a function of time

The degree of conversion of lactose into its hydrolysis products glucose and galactose was measured for capillaries produced according to Example 1, at a temperature of 70°C and a pressure of 2 atmospheres, with an axial flow of lactose solution of 300 ml/min. The level of the enzymatic hydrolysis was corrected for the spontaneous hydrolysis measured under comparable time and temperature conditions, corresponding to the experimental conditions for ultrafiltration in the absence of microorganisms. Measured spontaneous hydrolysis never exceeded 0.3%.

The initial net degree of conversion in the tests run under these conditions was approximately 18%, decreasing with time to a value of 5% after 250 hours.

Degree of conversion at steady state, as a function of pressure

This test was run under the same conditions indicated for the previous test, with the result that the degree of conversion is substantially inversely proportional to the applied pressure. As significative values, the degree of conversion was measured as 11% at 0.5 atmospheres, 6% at 2 atmospheres and 3% at 3 atmospheres.

Conclusions

The tests run show that the β-galactosidase is not affected by the process for permeabilizing and entrapping the cells in the capillary. In particular, the treatment with acetone to dehydrate and permeabilize the cytoplasmatic wall of the Caldariella acidophila increases the enzyme activity from 30 to 100 times with respect to fresh cells. Analogous results to those for β-galactosidase have been observed for the other enzyme activities measured for Calderiella acidophila. Finally, in general, an ultrafiltration device capable of giving rise to biocatalysis processes can be realized by using free enzymes or even microorganisms other than Caldariella acidophila, as long as this material is resistant to the treatment conditions used for the formation of the membranes in the form of hollow capillaries.

**Claims**

1. A biocatalytic capillary filter comprising a plurality of capillary hollow fibers formed of selective enzymatic semipermeable membrane characterised in that it consists essentially of a polymer matrix and entrapped dehydrated cells of thermophilic microorganism of the species Sulfolobus solfataricus DSM 1617, said fibers having been produced by a dry-wet spinning method wherein a polymer solution is processed, having a cell to polymer ratio of 1:2 to 1:1.

2. A filter as claimed in claim 1 in which said polymer is selected from polysulfone, cellulose acetate, aromatic polyamide and polyacrylonitrile.

3. A process for the manufacturing of a biocatalytic capillary filter comprising preparing a polymer solution in an anhydrous solvent, suspending in said polymer solution a charge consisting of dehydrated cells of the thermophilic microorganism Sulfolobus solfataricus DSM 1617, subjecting the resulting suspension to dry-wet spinning to prepare hollow capillaries with semipermeable membrane walls and mounting said capillaries in a filter device, in which said capillaries form the filtering wall.

4. A process as claimed in claim 3, wherein said polymer is selected from polysulfone, cellulose acetate, aromatic polyamide and polyacrylonitrile.

5. A process as claimed in claim 3, wherein said polymer is polysulfone and said solvent is dimethylacetamide.

6. A process as claimed in claim 3, wherein the ratio by weight of the microorganism cells to the polymer is 1:2 to 1:1.

7. Process for the hydrolysis of lactose to glucose and galactose, characterized by the fact that a substrate containing lactose at a temperature between 60 and 90°C is permeated through a filter as claimed in claim 1 and that the permeate containing glucose and galactose is collected.

**Patentansprüche**

1. Biokatalytischer Kapillarfilter, umfassend eine Mehrzahl von Kapillarhohlfasern, gebildet aus einer selektiven enzymatischen semipermeablen Membran, dadurch gekennzeichnet, daß er im wesentlichen aus einer Polymermatrix und eingeschlossenen dehydratisierten Zellen eines thermophilen Mikroorganismus der Species Sulfolobus solfataricus DSM 1617 besteht, wobei die Fasern durch ein Trocken-Naß-Spinnverfahren hergestellt worden sind, worin eine Polymerlösung behandelt wird, und ein Zellen zu Polymerverhältnis von 1:2 bis 1:1 besitzen.

2. Filter nach Anspruch 1, worin das Polymer aus Polysulfon, Celluloseacetat, aromatischem Polyamid und Polyacrylonitril gewählt wird.

3. Verfahren zur Herstellung eines biokatalytischen Kapillarfilters, bei dem eine Polymerlösung in einem wasserfreien Lösungsmittel hergestellt wird, in die Polymerlösung eine Beschickung, bestehend aus dehydratisierten Zellen des thermophilen Mikroorganismus Sulfolobus solfataricus DSM 1617, suspendiert wird, die erhaltene Suspension einem Trocken-Naß-Spinnen ausgesetzt wird, um hohle Kapillare mit semipermeablen Membranwänden herzustellen, und die Kapillare in eine Filtereinrichtung eingesetzt werden, worin die Kapillare die Filterwand bilden.

4. Verfahren nach Anspruch 3, worin das Polymer aus Polysulfon, Celluloseacetat, aromatischem Polyamid und Polyacrylonitril gewählt wird.

5. Verfahren nach Anspruch 3, worin das Polymer Polysulfon ist und das Lösungsmittel Dimethylacetamid ist.

6. Verfahren nach Anspruch 3, worin das Gewichtsverhältnis der Mikroorganismuszellen zu dem Polymer 1:2 bis 1:1 beträgt.

7. Verfahren zur Hydrolyse von Lactose zu Glucose und Galactose, dadurch gekennzeichnet, daß ein Substrat, das Lactose bei einer Temperatur zwischen 60 und 90°C enthält, durch einen Filter nach Anspruch 1 permeiert wird und daß das Permeat, das Glucose und Galactose enthält, gesammelt wird.

**Revendications**

1. Filtre capillaire biocatalytique comprenant de nombreuses fibres capillaries creuses formées d'une membrane enzymatique sélective semiperméable, caractérisé en ce qu'il est constitué essentiellement d'une matrice polymérique et de cellules déshydratées incorporées du microorganisme thermophile de l'espèce Sulfolobus solfataricus DSM 1617, les fibres ayant été produites par une méthode de filature au mouillé-sec (dry-wet spinning) dans laquelle est traitée une solution de polymère qui a un rapport de cellules à polymère de 1:2 à 1:1.

2. Filtre selon la revendication 1, où ledit polymère est choisi de polysulfone, acétate de cellulose, polyamide aromatique et polyacrylonitrile.

3. Procédé pour la fabrication d'une filtre capillaire biocatalytique comprenant les opérations des préparer une solution de polymère dans un solvant anhydre, suspendre dans cette solution de polymère une charge constituée de cellules déshydratées du microorganisme thermophile Sulfolobus solfataricus DSM 1617, soumettre la suspension résultante à filature au mouillé-sec (dry-wet spinning) pour préparer des capillaires creux avec parois à membrane semiperméable, et monter les capillaires dans un dispositif à filtre, où les capillaires forment la paroi filtrante.

4. Procédé selon la revendication 3, où le polymère est choisi de polysulfone, acétate de cellulose, polyamide aromatique et polyacrylonitrile.

5. Procédé selon la revendication 3, où ledit polymère est polysulfone et le solvant est diméthylacétamide.

6. Procédé selon la revendication 3, où le rapport en poids entre cellules de microorganisme et polymère est de 1:2 à 1:1.

7. Procédé pour l'hydrolyse de lactose à glucose et galactose caractérisé en ce qu'un substrat contenant du lactose à une température entre 60 et 90°C est perméé à travers un filtre tel que revendiqué dans la revendication 1 et le perméat contenant glucose et galactose est recueilli.